(19) Europäisches Patentamt
European Patent Office
Office européen des brevets

(11) Veröffentlichungsnummer: **0 214 371 A2**

(12) **EUROPÄISCHE PATENTANMELDUNG**

(21) Anmeldenummer: 86107160.3

(22) Anmeldetag: 27.05.86

(51) Int. Cl.[4]: C 07 C 91/26, C 01 B 33/28, C 07 C 1/20

(30) Priorität: 13.09.85 DE 3532748

(43) Veröffentlichungstag der Anmeldung: 18.03.87 Patentblatt 87/12

(84) Benannte Vertragsstaaten: BE DE FR GB SE

(71) Anmelder: Degussa Aktiengesellschaft
Weissfrauenstrasse 9
D-6000 Frankfurt am Main 1(DE)

(72) Erfinder: Baacke, Michael, Dr.
Grünaustrasse 19
D-6450 Hanau 9(DE)

(72) Erfinder: Kleinschmit, Peter, Dr.
Wildaustrasse 19
D-6450 Hanau 9(DE)

(54) **Templatverbindungen, ihre Herstellung und Verwendung.**

(57) Templat-Verbindungen der Formal

$$(HO\text{-}C_2H_4)_3\overset{\oplus}{N} - (CH_2)_n - \overset{\oplus}{N}(C_2H_4 - OH)_3\ 2\ Br^{\ominus},$$

wobei n = 3,4 oder 5 ist, werden hergestellt, indem man Triäthanolamin mit α,ω-Dibromalkanen, gegebenenfalls in Gegenwart eines Lösungsmittels umsetzt. Dabei kann man das α,ω-Dibromalkan zu dem vergelegten Triäthanolamin bei Reaktionstemperatur zutropfen und anschliessend das Reaktionsprodukt in bekannter Weise aufarbeiten.

Die Templat-Verbindungen werden zur Herstellung von Zeolithen des Pentasil-Typs eingesetzt.

Diese Zeolithe haben die Zusammensetzung:

$$(0,4\text{-}0,7)Na_2O/(0,3\text{-}0,6)Q_2O/Al_2O_3/(30\text{-}80)SiO_2/(4\text{-}10)H_2O$$

sowie das folgende Röntgenbeugungsdiagramm

| d Å | J/Jo |
|---|---|
| 11,0 ± 0,2 | 30 – 70 |
| 10,0 ± 0,2 | 30 – 80 |
| 4,4 ± 0,1 | 10 – 20 |
| 4,3 ± 0,1 | 10 – 20 |
| 3,85 ± 0,05 | 100 |
| 3,74 ± 0,05 | 40 – 65 |
| 3,66 ± 0,05 | 20 – 40 |
| 3,43 ± 0,03 | 15 – 25 |
| 2,01 ± 0,02 | 5 – 15 |
| 1,99 ± 0,02 | 5 – 15 |

Sie werden hergestellt, indem man eine wässrige Suspension von gefällter Kieselsäure mit wässriger Natriumaluminatlauge und einer wässrigen Lösung der Templatverbindung vermischt, diese Mischung bei autogenem Druck umsetzt, das Produkt trocknet und calciniert.

Die Zeolithen des Pentasiltyps werden bei der Umwandlung von Methanol in Kohlenwasserstoffe eingesetzt.

EP 0 214 371 A2

Degu 0214371

85 165 MS

Degussa Aktiengesellschaft
6000 Frankfurt am Main 1

# Templatverbindungen, ihre Herstellung und Verwendung

Die Erfindung betrifft Templatverbindungen der Formel
$(HO-C_2H_4)_3N^{\oplus}-(CH_2)_n-N^{\oplus}(C_2H_4-OH)_3\ 2\ Br^{\ominus}$
das Verfahren zu ihrer Herstellung, deren Verwendung zur Herstellung von Zeolithen des Pentasiltyp, die Herstellung der Zeolithen des Pentasiltyps, die Zeolithen des Pentasiltyps sowie deren Verwendung bei der Umwandlung von Methanol in Kohlenwasserstoffe.

Zeolithe vom Typ des Pentasils sind bekannt. Sie werden beschrieben von Doelle et al in Journal of Catalysies 71, 27-40 (1981).

Aus der EP-PS 42 226 sind Verbindungen der allgemeinen Formel

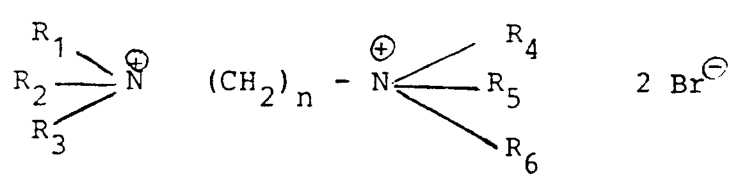

bei denen n = 3 bis 12 und $R_1$ bis $R_6$ gleich oder verschieden sein können, 1 bis 8 Kohlenstoffatome enthaltende Alkyl- oder Hydroxyalkylgruppen sein können und bis zu fünf der Gruppe $R_1$ bis $R_6$ Wasserstoff bedeuten können, bekannt.

Mit dieser Verbindung wird der Zeolith EU-1 hergestellt.

Der Zeolith EU-1 ist kein Zeolith des Pentasiltyps.

Aus der FR-PS 1 355 982 sind die Verbindungen

$$(HO-C_2H_4)_3 \overset{\oplus}{N} - (CH_2)_6 - \overset{\oplus}{N} (C_2H_4-OH)_3 \; 2\, Br^{\ominus}$$

und

$$(HO-C_2H_4)_3 \overset{\oplus}{N} - (CH_2)_2 - \overset{\oplus}{N} (C_2H_4-OH)_3 \; 2\, Br^{\ominus}$$

bekannt.

Nachteiligerweise kann mit diesen bekannten Verbindungen ebenfalls kein Zeolith des Pentasiltyps hergestellt werden.

Gegenstand der Erfindung sind die Verbindungen der Formel

$$(HOC_2H_4)_3 \overset{\oplus}{N} - (CH_2)_n - \overset{\oplus}{N} (C_2H_4 - OH)_3 \; 2\, Br^{\ominus},$$

wobei n = 3,4 oder 5 ist.

Diese Verbindung wird im folgenden auch als Templatverbindung bezeichnet.

Ein weiterer Gegenstand der Erfindung ist ein Verfahren zur Herstellung der Verbindungen der Formel

$$(HO-C_2H_4)_3 \overset{\oplus}{N} - (CH_2)_n - \overset{\oplus}{N} (C_2H_4-OH)_3 \; 2\, Br^{\ominus},$$

wobei n = 3,4 oder 5 ist, welches dadurch gekennzeichnet ist, daß man Triäthanolamin mit $\alpha,\omega$-Dibromalkanen, die 3,4 oder 5 C-Atome aufweisen, gegebenenfalls in Gegenwart eines Lösungsmittel, umsetzt, und anschließend das Reaktionsprodukt in bekannter Weise aufarbeitet.

In einer bevorzugten Ausführungsform kann man sowohl das Triäthanolamin als auch die $\alpha,\omega$-Dibromalkanverbindung gemeinsam in einem Lösungsmittels lösen und bei der Siedetemperatur des Lösungsmittel zur Umsetzung bringen. Insbesondere kann die Reaktion dann im Rückfluß durchgeführt werden.

Als Lösungsmittel können aliphatische Alkohole wie z.B. Ethanol, Methanol, Propanol und/oder deren Isomere, eingesetzt werden.

Wesentliche Voraussetzung für die Verwendung als Lösungsmittel ist, daß die Lösungsmittel die Reaktionspartner lösen und für den Ablauf der Reaktion ausreichend polar sind.

In einer anderen Ausführungsform der Erfindung kann man die Reaktion des Triäthanolamins mit dem $\alpha,\omega$-Dibromalkan in Abwesenheit eines Lösungsmittels durchführen, in dem man das Triäthanolamin vorlegt und das $\alpha,\omega$-Dibromalkan, vorzugsweise in stöchiometrischer Menge, bei Reaktionstemperatur, vorzugsweise bei 100 bis 115°C, jedoch nicht über 120°C, zutropfen läßt.

Ein weiterer Gegenstand der Erfindung ist die Verwendung der Verbindungen der Formel

$$(HO-C_2H_4)_3 \overset{\oplus}{N} - (CH_2)_n - \overset{\oplus}{N} (C_2H_4 - OH)_3 \; 2\,Br^{\ominus},$$

wobei n = 3,4 oder 5 ist, zur Herstellung von Zeolithen des Pentasil-Typs.

Der erfindungsgemäße Zeolith des Pentasiltyps ist gekennzeichnet durch

a) Summenformel

$(0,4-0,7)Na_2O/(0,3-0,6)Q_2O/Al_2O_3/(30-80)SiO_2/(4-10)H_2O$

b) Röntgenbeugungsdiagramm

| d (Å) | J/Jo |
|---|---|
| 11,0 ± 0,2 | 30 - 70 |
| 10,0 ± 0,2 | 30 - 80 |
| 4,4 ± 0,1 | 10 - 20 |
| 4,3 ± 0,1 | 10 - 20 |
| 3,85 ± 0,05 | 100 |
| 3,74 ± 0,05 | 40 - 65 |
| 3,66 ± 0,05 | 20 - 40 |
| 3,43 ± 0,03 | 15 - 25 |
| 2,01 ± 0,02 | 5 - 15 |
| 1,99 ± 0,02 | 5 - 15 |

Der erfindungsgemäße Zeolith des Pentasiltyps wird gemäß einem weiteren Gegenstand der Erfindung hergestellt, indem man gefällte Kieselsäure in Wasser suspendiert und zu einer Lösung von Natriumaluminat und NaOH in Wasser hinzugibt, eine Lösung von $(HO-C_2H_4)_3\overset{\oplus}{N}-(CH_2)_n-\overset{\oplus}{N}(C_2H_4-OH)_3\ 2\ Br^{\ominus}$, wobei n = 3,4 oder 5 ist, in Wasser zur Herstellung einer Reaktionsmischung mit der Zusammensetzung (1,0-4,0 Templatverbindung: (5-10) $Na_2O:Al_2O_3$: (30-90) $SiO_2$:(900-3000) $H_2O$, diese Reaktionsmischung unter autogenem Druck bei einer Temperatur von 100 bis 180 °C innerhalb eines Zeitraumes von 1 bis 20 Tagen umsetzt, das Produkt isoliert, trocknet und bei einer Temperatur von 300 bis 600°C calciniert.

Der erfindungsgemäße Zeolith des Pentasiltyps kann als Katalysator bei der Umwandlung von Methanol zu Kohlenwasserstoffen eingesetzt werden.

Beispiel 1

Zu 800 g Triethanolamin werden 615 g 1.5-Dibrompentan bei 110°C derart zugetropft, daß die Temperatur 120°C nicht übersteigt. Danach wird 8 Stunden bei einer Temperatur von 110°C nachgerührt. Anschliessend werden 1,5 l Wasser hinzugegeben. Das hochviskose Produkt ist vollständig wasserlöslich. Die wässrige Lösung kann direkt zur Herstellung von Zeolith verwendet werden. Die Analyse ergibt einen 100%igen Umsatz.

Beispiel 2

Zu 800 g Triethanolamin werden 577 g 1,4-Dibrombutan bei 110°C derart zugetropft, daß die Temperatur 120°C nicht übersteigt. Danach wird 8 Stunden bei einer Temperatur von 110°C nachgerührt. Anschliessend werden 1,5 l Wasser hinzugegeben. Das hochviskose Produkt ist vollständig wasserlöslich. Die wässrige Lösung kann direkt zur Herstellung von Zeolith verwendet werden. Die Analyse ergibt einen 100%igen Umsatz.

Beispiel 3

Zu 800 g Triethanolamin werden 540 g 1.3-Dibrompropan bei 110°C derart zugetropft, daß die Temperatur 120°C nicht übersteigt. Danach wird 8 Stunden bei einer Temperatur von 110°C nachgerührt. Anschliessend werden 1,5 l Wasser hinzugegeben. Das hochviskose Produkt ist vollständig wasserlöslich. Die wässrige Lösung kann direkt zur Herstellung von Zeolith verwendet werden. Die Analyse ergibt einen 100%igen Umsatz.

Beispiel 4

Zu 800 g Triethanolamin werden 652 g 1.6-Dibromhexan bei 110°C derart zugetropft, daß die Temperatur 120°C nicht übersteigt. Danach wird 8 Stunden bei einer Temperatur von 110°C nachgerührt. Anschliessend werden 1,5 l Wasser hinzugegeben. Das hochviskose Produkt ist vollständig wasserlöslich. Die wässrige Lösung kann direkt zur Herstellung von Zeolith verwendet werden. Die Analyse ergibt einen 100%igen Umsatz.

Beispiel 5

200 g Triethanolamin und 150 g 1,5-Dibrompentan werden in 1 l Ethanol gelöst und 48 h unter Rückfluß erhitzt. Der abgeschiedene Niederschlag wird abfiltriert und mit Ethanol gewaschen. Es werden 250 g Produkt (= 72,5 %) erhalten.

Beispiel 6

Eine Suspension von 100 g gefällter Kieselsäure in 700 ml Wasser wird zu einer Lösung von 5 g Natriumaluminat und 10 g NaOH in 75 ml Wasser gegeben. Dazu werden 60 g der wässrigen Lösung, die gemäß Beispiel 2 erhalten wurde, hinzugegeben. Diese Reaktionsmischung, die die Zusammensetzung 2,3 Templatverbindung nach Beispiel 2 :6,4 $Na_2O$ : 1 $Al_2O_3$ : 60 $SiO_2$: 1800 $H_2O$ aufweist, wird in einem Autoklaven bei 160°C unter Eigendruck 5 Tage gerührt. Nach dem Abkühlen wird das Produkt abfiltriert, getrocknet und 5 h bei 550°C calciniert. Das Röntgenbeugungsspektrum zeigt die charakteristischen Merkmale des Zeolithen vom Pentasil-Typ.

Die Analyse des isolierten, uncalcinierten Produktes (mit eingeschlossenem Kation) ist wie folgt:

-17-

0,91 % $Na_2O$
3,30 % $Al_2O_3$
87,7 % $SiO_2$
8,0 % GV (1100°C)
3,33 % C = 6,01 % Kation

Der Zeolith hat die folgende stöchiometrische Zusammensetzung: 0,45 $Na_2O$/0,48 $Q_2O/Al_2O_3$/45,2 $SiO_2$/ 5 $H_2O$, wobei Q ein Kation zum Ladungsausgleich zu $Al_2O_3$ ist.

Röntgenbeugungsdiagramm

| d Å | J/Jo |
|---|---|
| 11,14 | 40 |
| 9,99 | 30 |
| 4,42 | 13 |
| 4,32 | 13 |
| 3,86 | 100 |
| 3,74 | 46 |
| 3,67 | 27 |
| 3,46 | 16 |
| 2,01 | 7 |
| 1,99 | 7 |

Analyse (calciniertes Produkt)

0,93 % $Na_2O$
3,37 % $Al_2O_3$
89,54 % $SiO_2$
3,5 % Glühverlust
Oberfläche 430 m²/g

Beispiel 7

Eine Suspension von 300 g gefällter Kieselsäure in 2 l Wasser wird zu einer Lösung von 30 g Natriumaluminat und 45 g NaOH in 300 ml Wasser gegeben. Dazu werden 198 g der wässrigen Lösung, die gemäß Beispiel 3 erhalten wurde, hinzugegeben. Diese Reaktionsmischung, die die Zusammensetzung 1,3 Templatverbindung gemäß Beispiel 3:5,1 $Na_2O$ : 1 $Al_2O_3$ : 31 $SiO_2$: 920 $H_2O$ aufweist wird in einem Autoklaven bei 160°C unter Eigendruck 5 Tage gerührt. Nach dem Abkühlen wird das Produkt abfiltriert, getrocknet und 5 h bei 550°C calciniert. Das Röntgenbeugungsspektrum zeigt die charakteristischen Merkmale des Zeolithen vom Pentasil-Typ.

Analyse

1,77 % $Na_2O$
4,69 % $Al_2O_3$
82,4 % $SiO_2$
11,0 % Glühverlust (1100°C)
2,63 % C

Summenformel

0,62 $Na_2O$/0,32 $Q_2O$/$Al_2O_3$/30,0 $SiO_2$/4,5 $H_2O$, wobei Q ein Kation zum Ladungsausgleich von $Al_2O_3$ ist.

-11-

Röntgenbeugungsdiagramm

| d Å | J/Jo |
|---|---|
| 11,05 | 50 |
| 9,99 | 42 |
| 4,42 | 15 |
| 4,32 | 13 |
| 3,83 | 100 |
| 3,74 | 55 |
| 3,67 | 35 |
| 3,45 | 18 |
| 2,01 | 9 |
| 1,99 | 8 |

Beispiel 8

Eine Suspension von 300 g gefällter Kieselsäure in 2 l Wasser wird zu einer Lösung von 107g Natriumaluminat und 35 g NaOH in 300 ml Wasser gegeben. Dazu werden 182 g der wässrigen Lösung, die gemäß Beispiel 1 erhalten wurde, hinzugegeben. Diese Reaktionsmischung, die die Zusammensetzung 3,2 Templatverbindung gemäß Beispiel 1 :9,5 $Na_2O$ : 1 $Al_2O_3$ : 85 $SiO_2$ : 2550 $H_2O$ aufweist wird in einem Autoklaven bei 160°C unter Eigendruck 15 Tage gerührt. Nach dem Abkühlen wird das Produkt abfiltriert, getrocknet und 5 h bei 550°C calciniert. Das Röntgenbeugungsspektrum zeigt die charakteristischen Merkmale des Zeolithen vom Pentasil-Typ.

-12-

Beispiel 8

Analyse

0,60 % $Na_2O$
1,93 % $Al_2O_3$
89,1 % $SiO_2$
8,3 % Glühverlust (1100°C)
2,24 % C

Summenformel

$0{,}51\ Na_2O/0{,}58\ Q_2O/Al_2O_3/78{,}3\ SiO_2/7{,}3\ H_2O$, wobei Q ein Kation zum Ladungsausgleich von $Al_2O_3$ ist.

Röntgenbeugungsdiagramm

| d Å | J/Jo |
|---|---|
| 10,95 | 35 |
| 9,99 | 30 |
| 4,43 | 12 |
| 4,35 | 10 |
| 3,84 | 100 |
| 3,73 | 62 |
| 3,66 | 25 |
| 3,40 | 22 |
| 2,01 | 5 |
| 1,99 | 6 |

Beispiel 9

Eine Suspension von 200 g gefällter Kieselsäure in 1,5 l Wasser wird zu einer Lösung von 10 g Natriumaluminat und 20 g

-11-

NaOH in 50 ml Wasser gegeben. Dazu werden 124 g der wässrigen Lösung, die gemäß Beispiel 1 erhalten wurde, hinzugegeben. Diese Reaktionsmischung, die die Zusammensetzung 2,3 Templatverbindung gemäß Beispiel 1 :6,4 $Na_2O$ : 1 $Al_2O_3$ : 60 $SiO_2$ : 1800 $H_2O$ aufweist wird in einem Autoklaven bei 140°C unter Eigendruck 8 Tage gerührt. Nach dem Abkühlen wird das Produkt abfiltriert, getrocknet und 5 h bei 550°C calciniert. Das Röntgenbeugungsspektrum zeigt die charakteristischen Merkmale des Zeolithen vom Pentasil-Typ.

Beispiel 9

Analyse

0,93 % $Na_2O$
2,93 % $Al_2O_3$
85,8 % $SiO_2$
10,4 % Glühverlust (1100°C)
2,29 % C

Summenformel

0,52 $Na_2O$/o,39 $Q_2O$/$Al_2O_3$/49,7 $SiO_2$/8,6 $H_2O$, wobei Q ein Kation zum Ladungsaugleich von $Al_2O_3$ ist.

Röntgenbeugungsdiagramm

| d Å | J/Jo |
|---|---|
| 11,12 | 70 |
| 10,02 | 75 |
| 4,45 | 11 |
| 4,33 | 10 |
| 3,86 | 100 |
| 3,75 | 58 |

-11-

| | |
|---|---|
| 3,64 | 20 |
| 3,43 | 20 |
| 2,01 | 12 |
| 1,99 | 10 |

Beispiel 10 (Vergleichsbeispiel)

Eine Suspension von 100 g gefällter Kieselsäure in 750 ml Wasser wird zu einer Lösung von 5g Natriumaluminat und 10 g NaOH in 25 l Wasser gegeben. Dazu werden 90 g einer 50 %igen Lösung von $(HO-C_2H_4)_3\overset{\oplus}{N}-CH_2CH_2-\overset{\oplus}{N}(C_2H_4-OH)_3\ 2\ Br^{\ominus}$ in Wasser hinzugegeben. Diese Reaktionsmischung, die die Zusammensetzung 2,3 Templatverbindung 6,4 $Na_2O$ . 1 $Al_2O_3$ . 60 $SiO_2$ . 1800 $H_2O$ aufweist wird in einem Autoklaven bei 160°C unter Eigendruck 5 Tage gerührt. Nach dem Abkühlen wird das Produkt abfiltriert, getrocknet und 5 h bei 550°C calciniert. Das Röntgenbeugungsspektrum zeigt keine Linien (amorphes Produkt).

Beispiel 11

Der Zeolith des Pentasil-Typs, der nach Beispiel 8 hergestellt wird, wird in die katalytisch, aktive, saure Form überführt, indem man die Natrium-Form 2 h bei 80°C in wässriger 2N $H_2SO_4$-Lösung rührt, abfiltriert und bei 120°C trocknet.

Er ist durch die folgende Analyse gekennzeichnet:

Beispiel 11

Zeolith in der aktiven H-Form

Analyse

| | |
|---|---|
| 0,01 | % $Na_2O$ |
| 3,06 | % $Al_2O_3$ |
| 93,0 | % $SiO_2$ |
| 3,9 | % Glühverlust (1100°C) |

Summenformel

$0{,}005\ Na_2O/Al_2O_3/51{,}5\ SiO_2/7{,}2\ H_2O$

Der so erhaltene Zeolith wird verformt, indem man das Zeolithpulver mit Kieselsol (LUDOX HS 40) versetzt (0,7 ml Kieselsol pro Gramm Zeolith), die Masse durch ein Sieb (Maschenweite 1mm) drückt, 4 h bei 400°C calciniert und die Siebfraktion 0,5 bis 1,5 mm zur Umsetzung des Methanols auswählt.

Beispiel 12

Der verformte Zeolith gemäß Beispiel 11 wird zur Umwandlung von Methanol in Kohlenwasserstoffe eingesetzt. Dabei werden die folgenden Verfahrensparameter eingehalten:

| | |
|---|---|
| Temperatur | 375°C |
| Druck | 5 bar |
| Methanolpartialdruck | 2,2 bar |
| WHSV | 2,9 $h^{-1}$ |

Das Produkt weist die folgende Zusammensetzung nach Gewichtsprozent auf:

| | |
|---|---|
| Methan | 1,6 % |
| $C_2$-Kohlenwasserstoffe | 6,7 % |
| $C_3$-Kohlenwasserstoffe | 14,7 % |
| $C_4$-Kohlenwasserstoffe | 20,2 % |
| $C_5^+$-Kohlenwasserstoffe | 56,8 % davon 54 % Aromaten |

01 85 165 MS

Degussa Aktiengesellschaft
6000 Frankfurt am Main 1

Templatverbindungen, ihre Herstellung und Verwendung

Patentansprüche

1. Verbindungen der Formel

$$(HO\text{-}C_2H_4)_3\ N^{\oplus} - (CH_2)_n - N^{\oplus}\ (C_2H_4\text{-}OH)_3\ 2\ Br^{\ominus},$$

wobei n = 3,4 oder 5 ist.

2. Verfahren zur Herstellung der Verbindungen der Formel

$$(HO\text{-}C_2H_4)_3N^{\oplus}\text{-}(CH_2)_n\text{-}N^{\oplus}(C_2H_4\text{-}OH)_3\ 2\ Br^{\ominus}$$

wobei n = 3,4 oder 5 ist, gemäß Anspruch 1, dadurch gekennzeichnet, daß man Triäthanolamin mit α, ω-Dibromalkanen, die 3,4 oder 5 C-Atome aufweisen, gegebenenfalls in Gegenwart eines Lösungsmittel umsetzt, und anschließend das Reaktionsprodukt in bekannter Weise aufarbeitet.

3. Verwendung der Verbindungen der Formel

$$(HO\text{-}C_2H_4)_3N^{\oplus}\text{-}(CH_2)_n\text{-}N^{\oplus}(C_2H_4\text{-}OH)_3\ 2\ Br^{\ominus}$$

wobei n = 3,4 oder 5 ist, gemäß Anspruch 1 zur Herstellung von Zeolithen des Pentasil-Typs.

-2-

0-44918

4. Zeolith des Pentasiltyps, gekennzeichnet durch

a) Summenformel

$(0,4-0,7)Na_2O/(0,3-0,6)Q_2O/Al_2O_3/(30-80)SiO_2/(4-10)H_2O$

b) Röntgenbeugungsspektrum

| d (Å) | J/JO |
|---|---|
| 11,0 ± 0,2 | 30 - 70 |
| 10,0 ± 0,2 | 30 - 80 |
| 4,4 ± 0,1 | 10 - 20 |
| 4,3 ± 0,1 | 10 - 20 |
| 3,85 ± 0,05 | 100 |
| 3,74 ± 0,05 | 40 - 65 |
| 3,66 ± 0,05 | 20 - 40 |
| 3,43 ± 0,03 | 15 - 25 |
| 2,01 ± 0,02 | 5 - 15 |
| 1,99 ± 0,02 | 5 - 15 |

5. Verfahren zur Herstellung des Zeolithen des Pentasiltyps nach Anspruch 4, dadurch gekennzeichnet, daß man gefällte Kieselsäure in Wasser suspendiert und zu einer Lösung von Natriumaluminat und NaOH in Wasser hinzugibt, eine Lösung von $(HO-C_2H_4)_3N^{\oplus}-(CH_2)_n-n^{\oplus}(C_2H_4-OH)_3$ 2 $Br^{\ominus}$, wobei n = 3,4 oder 5 ist, in Wasser zur Herstellung einer Reaktionsmischung mit der Zusammensetzung (1,0-4.0)Templatverbindung: $(5-10)\ Na_2O:Al_2O_3:(30-90)\ SiO_2:(900-3000)\ H_2O$, diese Reaktionsmischung unter autogenem Druck bei einer Temperatur von 100 bis 180°C innerhalb eines Zeitraumes von 1 bis 20 Tagen umsetzt, das Produkt isoliert, trocknet und bei einer Temperatur von 300 bis 600°C calciniert.

6. Verwendung des Zeolithen des Pentasiltyps gemäß Anspruch 4 zur Herstellung von Kohlenwasserstoffen aus Methanol.